# EUROPÄISCHE PATENTSCHRIFT

(11) **EP 2 990 974 B1**
(45) Veröffentlichungstag und Bekanntmachung des Hinweises auf die Patenterteilung: **04.01.2023**
(21) Anmeldenummer: 15002067.5
(22) Anmeldetag: 10.07.2015
(51) Int. Cl.: G16H 40/40

(54) **BEATMUNGSGERÄT UND VERFAHREN FÜR EIN BEATMUNGSGERÄT**
ARTIFICIAL RESPIRATION DEVICE AND METHOD FOR SAME
APPAREIL DE RESPIRATION ET PROCEDE POUR UN APPAREIL DE RESPIRATION

(30) Priorität: 28.08.2014 DE 102014012793
(43) Veröffentlichungstag der Anmeldung: 02.03.2016
(73) Patentinhaber: Löwenstein Medical Technology S.A., 2557 Luxembourg (LU)
(72) Erfinder: BYCHKOV, Igor, 76137 Karlsruhe (DE); STOCKWALD, Florian, 76187 Karlsruhe (DE)
(74) Vertreter: Marx, Thomas

(56) Entgegenhaltungen:
- EP-A1- 1 659 489
- WO-A1-2009/023296
- WO-A1-2010/141922

## Beschreibung

Die vorliegende Erfindung betrifft ein Beatmungsgerät und ein Verfahren zum Betreiben eines Beatmungsgerätes. Das Beatmungsgerät umfasst wenigstens eine Rechnereinrichtung und wenigstens eine Gerätesoftware und wenigstens eine Gerätekonfiguration. Die Gültigkeiten der Computersoftware, der Gerätesoftware und der Gerätekonfiguration sind jeweils durch wenigstens einen Versionsstand charakterisiert. Zur Erkennung eines Versionskonfliktes werden die Versionsstände wenigstens teilweise miteinander abgeglichen.

Moderne Beatmungsgeräte werden in der Regel mit entsprechend angepassten Softwarelösungen betrieben. Dadurch können z. B. individuell einstellbare Beatmungsmodi oder eine automatische Erkennung von Atemstörungen besonders gut verwirklicht werden. So sind häufig eine Software für das Beatmungsgerät selbst und eine Software für einen externen Computer vorgesehen, von dem aus das Gerät ausgelesen und angesteuert werden kann. Zudem ist in den Geräten oft eine Konfigurationsdatei hinterlegt, welche z.B. die Sollwerte oder Grenzwerte für die zur Beatmung einzustellenden Beatmungsparameter vorgibt.

Die WO 2009/023296 A1 beschreibt ein Verfahren mit Prüfschritten zur Erkennung ob eine Gerätesoftware mit bestimmten Gerätetreibern kompatibel ist. Die WO 2009/023296 A1 beschreibt zudem das automatische Laden der benötigten Gerätetreiber.

Die EP 1 659 489 A1 beschreibt ein alternatives Verfahren mit Prüfschritten zur Erkennung ob eine Gerätesoftware mit bestimmten Gerätetreibern kompatibel ist.

Die WO 2010/141922 A1 beschreibt ein Softwareupdate bei medizinischen Geräten, wobei die Integrität der neuen Version der Software geprüft wird und der Nutzer informiert wird, wenn die Software nicht verifiziert werden konnte. Die Funktionalität des Gerätes, die dem Update entspricht, ist während des Updates nicht ausführbar, während andere Funktionalitäten lauffähig sind.

Um eine einwandfreie Funktion zu gewährleisten ist eine regelmäßige Aktualisierung der Software hilfreich bzw. notwendig. Allerdings kann es durch solche Aktualisierungen dazu kommen, dass inkompatible Versionsstände der einzelnen Softwareelemente vorliegen.

Solche inkompatiblen Versionsstände können dazu führen, dass bestimmte Funktionen nicht mehr einwandfrei funktionieren, wie z. B. das Einstellen oder das Auslesen des Beatmungsgerätes per PC-Software. Zudem kann das Aktualisieren von Software auf dem Beatmungsgerät oder dem Computer bewirken, dass bisherige Konfigurationen oder Beatmungseinstellungen nicht mehr verfügbar sind und neu erstellt werden müssen, was meistens sehr aufwendig ist. In sehr ungünstigen Fällen können inkompatible Versionsstände sogar die Bereitstellung der Beatmung nachteilig beeinflussen.

Es ist daher die Aufgabe der vorliegenden Erfindung, ein Beatmungsgerät und ein Verfahren zum Betreiben eines Beatmungsgerätes zur Verfügung zu stellen, mit denen unabhängig von Softwareaktualisierungen ein zuverlässiger Betrieb gewährleistet werden kann.

Diese Aufgabe wird gelöst durch ein Verfahren mit den Merkmalen des Anspruchs 1. Das erfindungsgemäße Beatmungsgerät ist Gegenstand des Anspruchs 8. Einige Vorteile und Merkmale der vorliegenden Erfindungen ergeben sich aus den Unteransprüchen. Weitere Vorteile und Merkmale sind in der allgemeinen Beschreibung und der Beschreibung des Ausführungsbeispiels angegeben.

Das erfindungsgemäße Verfahren dient zum Betreiben wenigstens eines Beatmungsgerätes. Das Beatmungsgerät umfasst wenigstens eine Rechnereinrichtung zur Steuerung einer Mehrzahl von Gerätefunktionen mit wenigstens einer Gerätesoftware und mit wenigstens einer Gerätekonfiguration mit Einstellparametern. Eine Gültigkeit der Gerätesoftware und eine Gültigkeit der Gerätekonfiguration sind jeweils durch wenigstens einen Versionsstand charakterisiert. Die Versionsstände werden zur Erkennung wenigstens eines Versionskonfliktes zwischen der Gerätesoftware und der Gerätekonfiguration miteinander verglichen werden. Dabei werden die Einstellparameter der Gerätekonfiguration ausgelesen, wenn ein ungültiger Versionsstand der Gerätekonfiguration und ein gültiger Versionsstand der Gerätesoftware vorliegen. Die Einstellparameter der Gerätekonfiguration werden in wenigstens eine erste Gruppe und wenigstens eine zweite Gruppe sortiert. Die erste Gruppe umfasst dabei von Versionskonflikt nicht betroffene Einstellparameter. Die zweite Gruppe umfasst Einstellparameter, welche vom Versionskonflikt betroffen sind. Dabei werden die Einstellparameter der ersten Gruppe beibehalten.

Das erfindungsgemäße Verfahren zum Betreiben des Beatmungsgerätes hat viele Vorteile. Ein erheblicher Vorteil ist, dass bei einem Vorliegen wenigstens eines ungültigen Versionsstandes die Einstellparameter der Gerätekonfiguration danach sortiert werden, ob sie vom Versionskonflikt betroffen sind oder nicht. Dadurch können die nicht betroffenen Einstellparameter trotz Vorliegen des Konfliktes verwendet werden, sodass damit der Betrieb fortgesetzt werden kann. Die Zuverlässigkeit und Sicherheit werden erheblich erhöht, da der Beatmungsbetrieb unabhängig von einer erfolgten oder vernachlässigten Softwareaktualisierung gewährleistet wird. Ein weiterer Vorteil ist, dass trotz eines Versionskonfliktes die Einstellparameter erhalten und nutzbar bleiben. Dadurch entfällt eine häufig zeitraubende Neueingabe der Einstellparameter. Besonders vorteilhaft ist zudem auch, dass auch eine Gerätesoftware bzw. eine Gerätekonfiguration mit einem ungültigen Versionsstand für den Betrieb des Beatmungsgerätes eingesetzt werden können. Das ist besonders hilfreich, wenn beispielsweise gerade nicht die Möglichkeit oder die Zeit besteht, eine Aktualisierung vorzunehmen.

Die Gültigkeit eines Versionsstandes ergibt sich insbesondere anhand wenigstens einer Aktualität. Insbesondere ist ein neuerer Versionsstand gültig. Ein älterer Versionsstand ist insbesondere ungültig. Insbesondere ist ein neuerer Versionsstand gegenüber einem älteren Versionsstand gültig. Möglich und bevorzugt ist aber auch, dass sich die Gültigkeit eines Versionsstandes anhand wenigstens einer zugeordneten Priorität ergibt. Insbesondere ist dabei ein Versionsstand mit einer höheren Priorität gegenüber einem Versionstand mit einer niedrigeren Priorität gültig.

Dabei wird die Priorität eines Versionsstandes bei einem Vergleich mit einem anderen Versionsstand z. B. höher gewichtet als eine Aktualität. So kann beispielsweise ein älterer Versionsstand mit einer höheren Priorität gegenüber einem neueren Versionsstand mit einer niedrigeren Priorität als gültig anerkannt werden. Der Versionsstand und insbesondere die Aktualität und/oder die Priorität sind vorzugsweise als wenigstens ein Programmelement in der Gerätesoftware und/oder der Gerätekonfiguration abgelegt und auslesbar. Die Prüfung der Versionsstände erfolgt vorzugsweise vor und/oder während einer Installation.

Die Gerätekonfiguration und die Gerätesoftware sind vorzugsweise in wenigstens einer Speichereinrichtung hinterlegt. Die Gerätekonfiguration und die Gerätesoftware können auch jeweils in einer separaten Speichereinrichtung abgelegt sein, z. B. einer Speicherkarte und einem fest installierten Speicher.

In Sinne dieser Erfindung sind die Einstellparameter insbesondere solche Werte, die die Einstellung bestimmter Geräteparamater festlegen. Geräteparameter sind dabei beispielsweise ein Druck, ein Flow, eine Drehzahl, eine Periodizität oder dergleichen. Der Einstellparameter gibt dabei insbesondere einen Wert oder Wertebereich für einen solchen Geräteparameter vor, z. B. einen Wert für einen Druck. Der Einstellparameter kann auch als wenigstens eine Funktion und/oder wenigstens ein Algorithmus ausgebildet sein. Ein Algorithmus als Einstellparameter beschreibt beispielsweise das Anheben und Absenken eines Druckes über die Zeit auf bestimmte Werte. Besonders bevorzugt geben die Einstellparameter Werte vor, mit denen die für die Beatmung einzustellenden Beatmungsparameter auf bestimmte Werte und/oder Verläufe festgelegt werden.

Die Einstellparameter der zweiten Gruppe sind insbesondere dadurch charakterisiert, dass sie bei einem vorgesehenen Betrieb von der Gerätesoftware abgefragt werden und in der Gerätekonfiguration nicht hinterlegt sind. Die Einstellparameter der zweiten Gruppe können auch dadurch charakterisiert sein, dass sie bei einem Betrieb nicht benötigt werden und von der Gerätsoftware nicht abgefragt werden. Vorzugsweise werden die nicht benötigten Einstellparameter nicht beibehalten. Die Einstellparameter der zweiten Gruppe sind insbesondere inkompatibel bei Vorliegen einer unzulässigen Kombination von Gerätesoftware und Gerätekonfiguration.

In einer besonders bevorzugten Weiterbildung werden die Einstellparameter der zweiten Gruppen in wenigstens eine erste Klasse und wenigstens eine zweite Klasse sortiert. Die erste Klasse umfasst dabei Einstellparameter, welche für einen vorgesehenen Betrieb des Beatmungsgerätes unkritisch sind. Die zweite Klasse umfasst insbesondere für einen vorgesehenen Betrieb kritische Einstellparameter.

Einstellparameter werden insbesondere dann als unkritisch definiert, wenn trotz des Versionskonfliktes wenigstens eine alternative Belegung möglich ist. Beispielsweise können unkritische Einstellparameter neu berechnet, übersprungen, gelöscht und/oder durch wenigstens einen anderen Einstellparameter ersetzt werden. Falls für einen unkritischen Einstellparameter keine alternative Belegung möglich ist, kann dennoch ein wenigstens teilweiser Betrieb möglich sein.

Kritische Einstellparameter sind insbesondere dadurch charakterisiert, dass sie für wenigstens eine Funktion des Beatmungsgerätes notwendig sind und diese Funktion bedingt durch den Versionskonflikt nicht einwandfrei bereitgestellt werden kann. Insbesondere ist ein kritischer Einstellparameter inkompatibel in Bezug zur Gerätesoftware.

Ein kritischer Einstellparameter muss insbesondere neu belegt werden, um kompatibel zur Gerätesoftware zu sein. Beispielsweise kann ein Einstellparameter kritisch sein, weil dieser einen Wert vorgibt, welcher mittels der Gerätesoftware und/oder des verwendeten Beatmungsgerätes nicht einstellbar ist. Dabei ist auch möglich, dass dessen Wert zwar einstellbar ist, die Gerätesoftware aber eine Programmkomponente enthält, welche die Einstellung eines solchen Wertes untersagt oder nicht zulässt.

Eine solche Einteilung der betroffenen Einstellparameter in eine kritische und unkritische Klasse ermöglicht eine zügige Bewertung des Versionskonfliktes und erleichtert daher die Berücksichtigung der notwendigen Schritte, um den Versionskonflikt wenigstens teilweise aufzuheben.

Vorzugsweise wird wenigstens ein Einstellparameter der ersten Klasse neu berechnet. Dabei wird für die Berechnung insbesondere wenigstens eine in der Gerätesoftware hinterlegte Vorschrift berücksichtigt. Beispielsweise kann die Gerätesoftware wenigstens einen Algorithmus umfassen, welcher zur Neuberechnung eines Einstellparameters herangezogen wird. Die Neuberechnung hat insbesondere zur Folge, dass der Einstellparameter trotz des Versionskonfliktes insbesondere für die Gerätekonfiguration übernommen bzw. verwendet werden kann.

Eine solche Neuberechnung hat den Vorteil, das auch neuere Geräte sowie Geräte mit neuerer Gerätesoftware mittels einer älteren Gerätekonfiguration eingestellt und betrieben werden können, obwohl die Gerätekonfiguration zunächst nicht alle notwendigen Einstellparameter für eine solche Kombination von Versionen bereitstellt. Die Vorschrift zur Neuberechnung kann dabei auch aus einer anderen Quelle als der Gerätesoftware stammen, beispielsweise aus einer externen Software oder der Gerätekonfiguration selbst.

Es ist möglich, dass die Vorschrift zur Berechnung wenigstens eine Berücksichtigung wenigstens eines Einstellparameters zugehörig zur ersten Gruppe vorsieht. Es kann auch eine Berücksichtigung eines Einstellparameters zugehörig zur zweiten Gruppe vorgesehen sein. Anhand der Berücksichtigung wird insbesondere wenigstens eine Querabhängigkeit wenigstens zweier Einstellparameter festgestellt.

Die Neuberechnung erfolgt dabei insbesondere anhand der querabhängigen Einstellparameter. Bevorzugt werden dabei bereits übernommene und/oder beibehaltene und/oder neu belegte Einstellparameter berücksichtigt. Kritische Einstellparameter werden insbesondere zur Neuberechnung anderer kritischer Einstellparameter nicht berücksichtigt. Durch das Heranziehen von Querabhängigkeiten wird die Anpassung kritischer Einstellparameter erheblich erleichtert.

In einer vorteilhaften Ausgestaltung wird wenigstens ein Einstellparameter der ersten Klasse anhand wenigstens einer Benutzereingabe eingestellt. Beispielsweise kann der Wert für den Einstellparameter direkt manuell eingegeben werden. Dem Benutzer können auch verschiedene Werte vorgeschlagen werden, aus denen er einen passenden auswählt. Möglich ist auch eine Übertragung von Einstellparametern aus einer anderen Gerätekonfiguration und/oder von einem externen Computer aus. Eine solche Benutzereingabe ermöglicht ein schnelles und unkompliziertes Ersetzen von Einstellparametern.

Besonders bevorzugt wird wenigstens ein Einstellparameter der ersten Klasse durch wenigstens eine Werkseinstellung ersetzt. Die Werkseinstellung ist vorzugsweise in wenigstens einer Speichereinrichtung hinterlegt. Möglich ist auch eine Übernahme von großen Teilen und/oder einer kompletten Werkseinstellung. Dabei kann die Werkseinstellung auch eine Mehrzahl und/oder alle Einstellparameter der Klassen ersetzen.

In besonderen Fällen können auch kritische Einstellparameter der zweiten Klasse durch wenigstens eine Werkseinstellung ersetzt werden. Dabei kann vorgesehen sein, dass die Werkseinstellung einen nur teilweise ausreichenden Ersatz für den kritischen Einstellparameter bietet. Bei einer unzureichenden Belegung des kritischen Einstellparameters kann beispielsweise ein Betrieb in einem abgesicherten Modus und/oder ein Notbetrieb vorgesehen sein. Dabei wird vorzugsweise wenigstens ein Hinweis an den Benutzer ausgegeben. Ein Ersetzen von Einstellparametern durch entsprechende Werkseinstellungen bietet eine zuverlässige Möglichkeit, die bei einem Versionskonflikt auftretenden Inkompatibilitäten schnell und einfach zu bereinigen.

Es ist möglich und bevorzugt, dass bei einem Vorliegen wenigstens eines Einstellparameters der zweiten Klasse wenigstens ein Hinweis über den Versionskonflikt an den Benutzer ausgegeben wird. Der Hinweis kann beispielsweise eine Aufforderung umfassen, die Gerätesoftware und/oder die Gerätekonfiguration zu aktualisieren. Dabei kann eine gültige Kombination vorgeschlagen werden. Möglich ist auch, dass eine Bezugsquelle genannt wird und/oder eine Verbindung zu einer Bezugsquelle aufgebaut wird, z. B. über das Internet. Es ist möglich, dass bis zur Aktualisierung der Betrieb angehalten oder eingeschränkt ausgeführt wird.

Möglich und bevorzugt ist auch, dass bei einem Vorliegen wenigstens eines Einstellparametes der zweiten Klasse wenigstens eine Benutzereingabe eingefordert wird. Insbesondere wird nach der Benutzereingabe ein vorheriger und insbesondere gültiger Versionsstand der Gerätesoftware wiederhergestellt. Möglich ist auch, dass ein vorheriger und gültiger Versionsstand der Gerätekonfiguration und/oder einer externen Software wiederhergestellt wird. Vorzugsweise ist der vorherige Versionsstand in wenigstens einer Speichereinrichtung abgelegt.

Beispielsweise wird vor der Durchführung einer Softwareaktualisierung und/oder einer Aktualisierung der Gerätekonfiguration wenigstens ein Backup erstellt. Durch die Benutzereingabe wird beispielsweise ein Aktualisierungsvorgang abgebrochen, während dieser bereits begonnen haben kann. Besonders bevorzugt erfolgt die Erkennung und Prüfung der Versionsstände vor einer Aktualisierung und insbesondere vor einer Installation einer neuen Software oder Konfiguration.

Bei einem Vorliegen wenigstens eines Einstellparameters der zweiten Klasse kann auch wenigstens eine Benutzereingabe eingefordert werden, welche einen Betrieb mit einem ungültigen Versionsstand von Gerätesoftware und Gerätekonfiguration frei gibt. Insbesondere wird dabei nach der Benutzereingabe der Betrieb mit dem ungültigen Versionsstand in einem wenigstens teilweise eingeschränkten Modus fortgesetzt. Beispielsweise kann nur ein Teil der Gerätefunktionen nutzbar sein und/oder der Betrieb wird mit Einstellparametern fortgesetzt, welche nur teilweise den vom Benutzer gewünschten entsprechen.

Es ist möglich, dass für einen solchen eingeschränkten Modus wenigstens ein Einstellparameter in einer Speicheinrichtung hinterlegt ist, durch den kritische Einstellparameter und/oder fehlende Einstellparameter ergänzt bzw. neu belegt werden. Eine solche Ausgestaltung bietet auch dann eine Nutzung des Gerätes, wenn aufgrund fehlender und/oder unzureichender Aktualisierungen Versionskonflikte vorliegen.

Das Beatmungsgerät weist wenigstens eine Rechnereinrichtung zur Steuerung einer Mehrzahl von Gerätefunktionen auf. Die Rechnereinrichtung umfasst wenigstens eine Gerätesoftware und wenigstens eine Gerätekonfiguration mit Einstellparametern. Eine Gültigkeit der Computersoftware und eine Gültigkeit der Gerätesoftware sowie eine Gültigkeit der Gerätekonfiguration sind jeweils durch wenigstens einen Versionsstand charakterisiert. Zur Erkennung eines Versionskonfliktes werden die Versionsstände wenigstens teilweise miteinander verglichen. Dabei wird von der Gerätesoftware wenigstens eine Programmkomponente für die Computersoftware bereitgestellt, wenn ein ungültiger Versionsstand der Computersoftware und ein gültiger Versionsstand der Gerätesoftware erkannt wird. Die Programmkomponente umfasst dabei wenigstens einen Grenzwert. Zudem umfasst die Programmkomponente wenigstens eine Plausibilitätsregel. Anhand der Plausibilitätsregel und des Grenzwertes kann wenigstens ein Einstellparameter in der Gerätekonfiguration von der Computersoftware ausgehend eingestellt werden.

Mit einer solchen Kombination kann der Betrieb des Beatmungsgerätes völlig unabhängig von Versionsständen der Computersoftware und der Gerätesoftwäre sowie der Gerätekonfiguration aufrechterhalten und eine Beatmung gewährleistet werden.

Beispielsweise kann somit auch ein Computer mit einer veralteten und ungültigen Computersoftware zur Ansteuerung oder zum Auslesen eines Beatmungsgerätes mit einer neuen und gültigen Gerätesoftware oder Gerätekonfiguration eingesetzt werden. Mittels der Programmkomponente können von der ungültigen Computersoftware aus auch solche Einstellparameter beeinflusst werden, für die der Versionsstand der Computersoftware nicht ausgelegt ist.

Insbesondere ist für wenigstens einen Einstellparameter wenigstens ein Grenzwert hinterlegt. Insbesondere werden die vom Versionskonflikt betroffenen Einstellparameter und beispielsweise unkritische und/oder kritische Einstellparameter anhand des Grenzwertes angepasst. Es ist möglich, dass auch ein ungültiger Versionsstand der Gerätekonfiguration vorliegt.

Dabei kann auch eine Anpassung der Gerätekonfiguration vorgesehen sein, beispielsweise wie sie zuvor für das Verfahren zum Betreiben des Beatmungsgerätes beschrieben wurde. Die Gerätekonfiguration kann auch gültig sein. Es kann vorgesehen sein, dass trotz der bereitgestellten Programmkomponente ein wenigstens teilweise eingeschränkter Betrieb der Computersoftware möglich ist. Beispielsweise kann von der Computersoftware aus auf nur einen Teil der Funktionen des Beatmungsgerätes zugegriffen werden. Möglich ist auch, dass nur auf einen Teil der Gerätekonfiguration von der Computersoftware aus Einfluss genommen werden kann.

In einer bevorzugten Weiterbildung wird durch die Programmkomponente wenigstens eine Querabhängigkeit zwischen wenigstens zwei Einstellparametern definiert. Möglich ist auch eine Querabhängigkeit zwischen drei oder vier oder einer Mehrzahl von Einstellparametern. Das verbessert die Genauigkeit einer Neubelegung von Einstellparametern erheblich.

In einer besonders bevorzugten Weiterbildung wird durch die Programmkomponente wenigstens ein Programmcode bereitgestellt. Dabei wird der Programmcode vorzugsweise in wenigstens einer von der Gerätesoftware und der Computersoftware gemeinsam genutzten Programmbibliothek abgelegt. Das hat den Vorteil, dass sowohl die Gerätesoftware als auch die Computersoftware gleichzeitig auf die Programmkomponente zugreifen und/oder diese beeinflussen können. Ein weiterer Vorteil ist, dass sich eine Änderung der Programmkomponente durch die Gerätesoftware auch sofort auf die Computersoftware auswirkt und umkehrt.

Der Programmcode wird besonders bevorzugt durch wenigstens eine ausführbare Datei bereitgestellt. Insbesondere wird der Programmcode durch eine DLL-Datei und/oder eine vergleichbare geeignete Datei breitgestellt. Eine solche Ausgestaltung ist besonders gut geeignet, um für die ungültige Computersoftware die Programmkomponenten bereitzustellen, welche diese für eine einwandfreie Kommunikation mit der Gerätesoftware benötigt.

Die Computersoftware ist vorzugsweise wenigstens teilweise rückwärts kompatibel ausgestaltet. Insbesondere ist die Computersoftware dazu geeignet und ausgebildet, von wenigstens einem als ungültigen erkannten Versionsstand einer Gerätesoftware betrieben zu werden und/oder eine solche zu betreiben. Insbesondere ist die Computersoftware so ausgestaltet, dass sie mit wenigstens einer ungültigen und insbesondere älteren Version einer Gerätesoftware und/oder einer Gerätekonfiguration in vorgesehener Weise betreibbar ist. Eine solche Ausgestaltung hat den Vorteil, das auch ältere Beatmungsgeräte von neueren Computern aus eingestellt und oder ausgelesen werden können.

Die Verfahren können auch dahingehend ausgebildet sein, dass mittels wenigstens eines Masterprogramms eine parallele Einstellung von einer Vielzahl von Gerätekonfigurationen vorgenommen werden kann. Dabei ist das Masterprogramm insbesondere nur bei gültigen Versionsständen ausführbar. Insbesondere betreffen die Gerätekonfigurationen dabei unterschiedliche Beatmungsgeräte. Ein solches Masterprogramm hat den Vorteil, dass darüber eine einfache und unkomplizierte Einstellung der Gerätekonfigurationen für mehrere Beatmungsgeräte erfolgen kann. Da hierbei in der Regel mehrere Beatmungsgeräte betroffen sind und häufig auch für die Beatmung entscheidende Einstellungen vorgenommen werden, bringt die an gültige Versionsstände gekoppelte Programmausführung ein hohes Maß an Sicherheit mit sich.

Die Verfahren können auch wenigstens ein Hilfsprogramm vorsehen, welches zur insbesondere programmbasierten Einstellung wenigstens einer Gerätekonfiguration dient. Insbesondere wird durch das Hilfsprogramm wenigstens eine Vorschrift zu einer Einstellung wenigstens eins Einstellparameters definiert.

Das Hilfsprogramm kann beispielsweise eine Vorschrift enthalten, welche Einstellparameter übernommen und/oder neu eingestellt und /oder gelöscht werden sollen. Die Vorschrift kann auch Grenzwerte zur Einstellung von Einstellparametern enthalten. Es ist möglich, dass das Hilfsprogramm bzw. dessen Ausführbarkeit an eine bestimmte Kombination von Versionsständen gekoppelt ist. Ein solches Hilfsprogramm hat den Vorteil, dass nach einer Aktualisierung oder bei Vorliegen von Versionskonflikten die Einstellparameter in den Gerätekonfigurationen automatisiert angepasst werden können.

Das erfindungsgemäße Beatmungsgerät umfasst wenigstens eine Rechnereinrichtung zur Steuerung einer Mehrzahl von Gerätefunktionen. Die Rechnereinrichtung weist wenigstens eine Gerätesoftware und wenigstens eine Gerätekonfiguration mit Einstellparametern auf. Eine Gültigkeit der Gerätesoftware und eine Gültigkeit der Gerätekonfiguration sind jeweils durch wenigstens einen Versionsstand charakterisiert. Die Rechnereinrichtung ist dazu geeignet und ausgebildet, die Versionsstände zur Erkennung eines Versionskonfliktes zwischen der Gerätesoftware und der Gerätekonfiguration wenigstens teilweise miteinander zu vergleichen. Dabei ist die Rechnereinrichtung dazu geeignet und ausgebildet, wenigstens einen ungültigen Versionsstand der Gerätekonfiguration und wenigstens einen gültigen Versionsstand der Gerätesoftware zu registrieren. Die Rechnereinrichtung ist zudem dazu geeignet und ausgebildet, die Einstellparameter der Gerätekonfiguration auszulesen und in wenigstens eine erste Gruppe mit vom Versionskonflikt nicht betroffenen und wenigstens eine zweite Gruppe mit vom Versionskonflikt betroffenen Einstellparametern zu sortieren. Die Rechnereinrichtung ist dabei dazu geeignet und ausgebildet, die Einstellparameter der ersten Gruppe beizubehalten.

Besonders bevorzugt ist das Beatmungsgerät so ausgebildet, dass es nach dem erfindungsgemäßen Verfahren sowie dessen Weiterbildungen betreibbar ist. Dadurch kann das Verfahren besonders gut umgesetzt und dessen Vorteile genutzt werden.

Weitere Vorteile und Merkmale der vorliegenden Erfindung ergeben sich aus der Beschreibung des Ausführungsbeispiels, welches mit Bezug auf die beiliegende Figur im Folgenden erläutert wird.

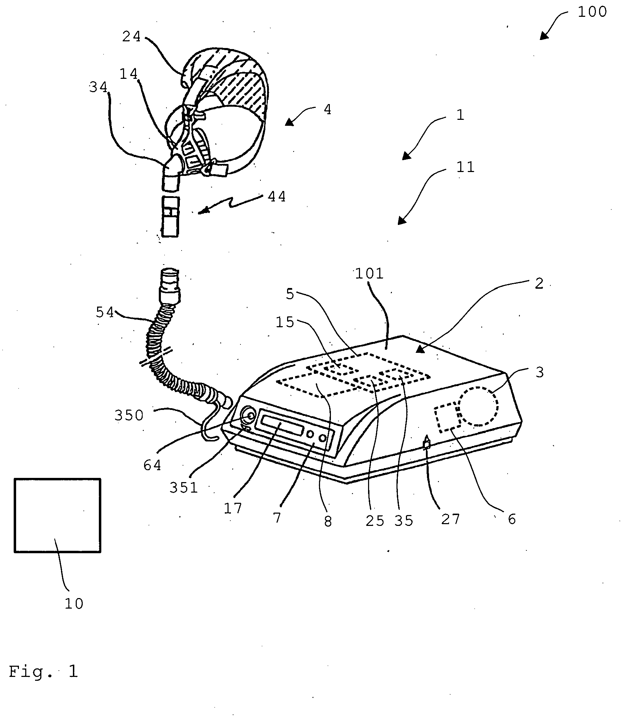

In der Figur 1 ist ein erfindungsgemäßes Beatmungsgerät 1 dargestellt.

In der Figur 1 ist ein Beatmungssystem 100 mit einem Beatmungsgerät 1 und einem stark schematisiert dargestellten externen Computer 10 gezeigt. Das Beatmungsgerät 1 ist bevorzugt ein Heimbeatmungsgerät 11. Das Beatmungssystem 100 und das Beatmungsgerät 1 sind hier zur Durchführung der erfindungsgemäßen Verfahren vorgesehen und ausgebildet.

Das Beatmungsgerät 1 umfasst eine von einem Gehäuse 101 umgegebene Beatmungseinrichtung 2 mit einer Gebläseeinrichtung 3 zur Erzeugung eines Luftstromes für die Beatmung. Zur Steuerung der Beatmungseinrichtung 2 ist eine Überwachungseinrichtung 5 mit einer Speichereinrichtung 15, einem Controller 25 und einer Sensoreinrichtung 35 vorgesehen.

Die Bedienung und Einstellung des Beatmungsgerätes 1 erfolgt über eine Bedieneinrichtung 7 mit einer Anzeigeeinrichtung 17 und einer Schnittstelle für den Datenaustausch 27. Zur Erhöhung des Feuchtegehaltes der Atemluft ist eine Befeuchteeinrichtung 6 vorgesehen.

Das Beatmungsgerät 1 weist eine Atemschnittstelle 4 auf, um den Luftstrom einem Benutzer zur Beatmung zuzuführen. Die Atemschnittstelle ist vorzugsweise als ein bei Beatmungsgeräten übliches Patienteninterface ausgebildet und beispielsweise als eine Vollgesichtsmaske oder als ein Nasal-Pillow oder als ein Tubus oder als eine Larynxmaske ausgestaltet sein. Die hier gezeigte Atemschnittstelle 4 ist eine als Nasalmaske ausgebildete Atemmaske 14. Zur Fixierung der Atemmaske 14 ist eine Kopfhaube 24 vorgesehen.

Zur Verbindung der Atemschnittstelle 4 mit der Beatmungseinrichtung 2 ist ein Verbindungsschlauch 54 vorgesehen, der mittels einer Koppeleinrichtung 64 mit der Beatmungseinrichtung 2 verbunden wird. Über ein Kuppelungselement 34 wird der Verbindungsschlauch 54 mit der Atemschnittstelle 4 verbunden. Zwischen dem Verbindungschlauch 54 und dem Kuppelungselement 12 ist ein Ausatmungselement 44 angeordnet, welches ein Ventil umfasst oder als ein solches ausgebildet ist. Das Ausatmungselement 44 ist insbesondere dazu vorgesehen, während der Ausatmung des Benutzers Rückatmen in das Beatmungsgerät 1 zu verhindern.

Die Überwachungseinrichtung 5 mit der Sensoreinrichtung 35 wirkverbunden, welche einen oder mehrere Sensoren zur Erfassung von Größen, welche charakteristisch für den Beatmungsparameter sind, aufweist. Beispielhaft hat die Sensoreinrichtung 35 einen hier nicht gezeigten Drucksensor, welcher die Druckverhältnisse im Bereich der Atemschnittstelle erfasst. Dazu ist der Drucksensor über einen Druckmessschlauch 350 mit der Atemschnittstelle 4 strömungsverbunden.

Der Druckmessschlauch 350 erstreckt sich dabei von der Atemschnittstelle 4 entlang der Verbindungsschlauches 54 zu einem Eingangsstutzen 351 am Gehäuse 101. Über den Eingangsstutzen 351 ist der Druckmessschlauch 350 mit dem Drucksensor der Sensoreinrichtung 35 strömungsverbunden.

Die Flowmessung erfolgt dabei über eine Drossel, welche im Hauptkanal der Strömungsverbindung zwischen Gebläseeinrichtung und Atemschnittstelle 4 angeordnet ist. Möglich ist auch eine Anordnung in einem Nebenkanal. Um weitere Beatmungsparameter überwachen zu können, kann die Sensoreinrichtung 35 auch mit Sensoren zur Messung der Atemexkursion, zur Messung einer Sauerstoffsättigung des Blutes und/oder zur Messung einer EEG-, einer EMG-, einer EOG- oder einer EKG-Aktivität ausgestattet sein.

Des Weiteren weist die Überwachungseinrichtung 5 einen Controller 25 zur Ansteuerung der Gebläseeinrichtung 3 auf. Der Controller 25 stellt einen notwendigen Minimaldruck bereit und kompensiert Druckschwankungen, die durch die Atemtätigkeit des Benutzers bedingt sind. Beispielsweise erfasst der Controller 25 auch den gegenwärtigen Druck in der Atemmaske 14 und regelt die Leistung der Gebläseeinrichtung 3 entsprechend nach, bis ein gewünschter Beatmungsdruck anliegt.

Das hier gezeigte Beatmungsgerät 1 kann als ein Fix-Level-Gerät oder auch als ein Automatic-Level-Gerät ausgebildet sein. Insbesondere erfolgt dabei durch die Überwachungseinrichtung 5 eine Regelung auf Sollwerte, welche zuvor anhand der charakteristischen Atmung eines Benutzers individuell berechnet und festgelegt worden sind.

Es ist auch möglich, das die Beatmungseinrichtung 2 dynamisch und insbesondere je nach Atemphase des Benutzers angepasst wird. Beispielsweise kann anhand der Überwachungseinrichtung 5 ein Atemphasenwechsel erkannt werden, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z.B. Schnarchen, Atemabflachungen und/oder obstruktive Druckspitzen mit entsprechenden Einstellungen der Beatmungsparameter.

Zum Ansprechen der Gerätekomponenten und zum Steuern der insbesondere von diesen Gerätekomponenten bereitgestellten Gerätefunktionen weist das Beatmungsgerät 1 hier eine Rechnereinrichtung 8 auf. Die Gerätekomponenten sind z. B. die Beatmungseinrichtung 2, die Gebläseeinrichtung 3, die Überwachungseinrichtung 5, die Befeuchtereinrichtung 6, die Bedieneinrichtung 7, die Speichereinrichtung 15, die Anzeigeeinrichtung 17, der Controller 25, die Schnittstelle 27 und die Sensoreinrichtung 35 sowie weitere hier nicht gezeigte Gerätekomponenten.

Die Rechnereinrichtung 8 ist hier als ein Mikrocomputer ausgebildet und umfasst neben einem Betriebssystem eine Gerätesoftware. Mit der Rechnereinrichtung 8 können Daten mit dem externen Computer 10 ausgetauscht werden. Dabei werden die Rechnereinrichtung 8 und der Computer 10 über die Schnittstelle 27 des Beatmungsgerätes 1 miteinander verbunden. Die Schnittstelle 27 kann dabei auch zur drahtlosen Kommunikation ausgebildet sein.

Von dem externen Computer aus können eine Vielzahl von Gerätefunktionen bedient und eingestellt werden. Dazu weist der Computer 10 eine Computersoftware mit einer Benutzeroberfläche auf, mittels der einfach und übersichtlich Einstellungen vorgenommen werden können. Mittels der Computersoftware können auch Daten aus dem Beatmungsgerät 1 abgerufen werden, wie z. B. eine Beatmungsstatistik, Fehlermeldungen, aktuelle Einstellungen oder andere in der Speichereinrichtung 25 oder der Recheneinrichtung 8 abgelegte Daten. Die Computersoftware umfasst z. B. auch ein Programm zur Auswertung der Beatmungsstatistik.

Zur Anpassung der Gerätesoftware an das Beatmungsgerät 1 ist eine Gerätekonfiguration hinterlegt. Die Gerätekonfiguration kann beispielsweise eine oder mehrere Dateien umfassen und in der Speichereinrichtung 15 oder auf einer Speicherkarte, welche in das Gerät 1 eingeschoben wird, abgelegt sein. Die Gerätekonfiguration umfasst eine Vielzahl von Einstellparametern, nach denen die einzelnen Gerätefunktionen und z. B. die Beatmungseinrichtung 2 konfiguriert und betrieben werden.

Zur Erkennung von Versionskonflikten werden die Versionsstände der Computersoftware und der Gerätesoftware sowie der Gerätekonfiguration, nachfolgend auch als Betriebsprogramme bezeichnet, miteinander abgeglichen. Das kann beispielsweise beim Hochfahren des Computers 100 oder beim Einschalten des Beatmungsgerätes 1 oder auch nach bzw. vor einem Update erfolgen.

Um trotz unterschiedlicher Versionsstände eine Kompatibilität der Betriebsprogramme untereinander aufrechtzuerhalten und somit einen zuverlässigen Betrieb des Beatmungsgerätes 1 zu gewährleisten, sind erfindungsgemäß eine Reihe von Maßnahmen vorgesehen, von denen hier einige beispielhaft vorgestellt werden.

Beispielsweise wurde die Gerätesoftware aktualisiert und bei einem Gerät 1 mit einer alten und daher ungültigen Gerätekonfiguration eingesetzt. Beispielsweise kann auch eine neue Gerätesoftware für ein noch nicht unterstütztes Gerät 1 eingesetzt werden. In derartigen Fällen muss das Gerät 1 dann mit einer Gerätekonfiguration eingestellt werden, die aus einem älteren Geräte- oder Softwarestand kommt.

Zur Aufrechterhaltung der Kompatibilität der Betriebsprogramme werden die in der Gerätekonfiguration hinterlegten Einstellparameter ausgelesen und danach sortiert, ob sie vom Versionskonflikt betroffen sind oder nicht. Die durch den Versionskonflikt nicht betroffenen Einstellparameter werden unverändert übernommen.

Anschließend werden die verbliebenen Einstellparameter danach eingeteilt, ob sie den Betrieb des Beatmungsgerätes 1 unkritisch oder kritisch beeinflussen können. Neu hinzugekommene, unkritische Einstellparameter werden dann sinnvoll belegt in der Form, dass entweder Werkseinstellungen angenommen werden oder aus den anderen Einstellparametern logisch sinnvolle Parameterwerte ermittelt werden. Kritische Einstellparameter, die inkompatibel werden oder neu hinzukommen, führen entweder dazu, dass der Benutzer die Abhängigkeit auflösen muss oder dass die Gesamtkonfiguration nicht übernommen wird.

Eine andere beispielhafte Maßnahme kann bei Vorliegen einer alten und somit ungültigen Konfiguration und einer neuen, aktualisierten Gerätesoftware ergriffen werden. Im Fall eines Updates der Gerätesoftware wird vor dem Update überprüft, ob mit der neuen Version die von der Gerätekonfiguration vorgegebenen Einstellungen in geeigneter Weise übernommen werden können. Ist dies nicht in gewünschter Weise der Fall, wird eine entsprechende Mitteilung an den Benutzer generiert. Ist keine geeignete Übernahme möglich, hat der Benutzer die Möglichkeit, den Updateprozess abzubrechen. Alternativ kann er das Update durchführen, wobei das Gerät 1 dann die Werkseinstellungen verwendet. Ist eine Übernahme möglich, erfordert aber Entscheidungen des Anwenders für einzelne Konfigurationseinstellungen, so können diese ebenfalls abgefragt werden.

Für bestimmte Einstellparameter oder Untermengen von Einstellparametern kann die Gerätesoftwäre für die Überführung von einer ungültigen in eine gültige, neue Konfiguration Vorgaben machen. Diese Vorgaben können dabei für bestimmte Kombinationen von Versionsständen der Betriebsprogramme vorgesehen sein. Dadurch wird die Übernahme einer neuen Konfiguration erheblich beschleunigt und vereinfacht. Z. B. kann eine solche Vorgabe angeben, welche Einstellparameter übernommen und welche nicht übernommen werden. So können beispielsweise Beatmungsparameter wie Solldruck übernommen werden, Komfortparameter, wie Displayhelligkeit aber nicht oder umgekehrt.

Beispielsweise kann eine ungültige Gerätekonfiguration einen Einstellparameter mit einem Wert von 27 hPa aufweisen. In einer neuen, gültigen Gerätekonfiguration kann der Einstellparameter dann z. B. einen Wertebereich von 4 bis 25 hPa umfassen. Die Übernahme des Einstellparameters in die gültige Konfiguration erfolgt dann beispielsweise anhand eines automatischen Zurücksetzens auf einen Wert von 25 hPa.

Die zuvor vorgestellten Maßnahmen ermöglichen einen Übertrag von einer Gerätekonfiguration, die in einer ersten Gerätesoftwareversion gültig war, in eine neue Gerätekonfiguration, die dann in einer zweiten, aktuelleren Gerätesoftwareversion gültig ist. Der Übertrag geschieht dabei über wenigstens einen Regelsatz, der Querabhängigkeiten und Prioritäten sowie Grenzwerte und Plausibilitätsregeln oder andere geeignete Anpassungsinstrumente berücksichtigt.

Dadurch können Updates für die Gerätesoftware eingespielt werden, ohne dass die bisherigen Einstellungen unsinnig oder ungültig werden oder gar das Gerät 1 in einen undefinierten Zustand gerät oder komplett neu konfiguriert werden muss. Durch solche Maßnahmen ist zudem gesichert, dass Gerätekonfigurationen auf unterschiedlichen Beatmungsgeräten 1 verwendet werden können, wenn diese Konfigurationen mit einem alten Softwarestand erstellt und beispielsweise per Datenträger versandt wurden.

Das Verfahren ermöglicht auch eine Bedienung von neueren Beatmungsgeräten 1 mit Computern 100, welche ältere und somit ungültige Versionsstände der Computersoftware aufweisen. Bei Vorliegen einer gültigen Gerätesoftware und einer ungültigen Computersoftware liefert die Gerätesoftware beispielsweise eine Programmkomponente, mit der sich die Computersoftware erweitern bzw. updaten kann, wodurch die Unterstützung des Beatmungsgerätes von der älteren Computersoftware aus ermöglicht wird. Die Programmkomponente kann beispielsweise über eine Speicherkarte, über einen USB-Massenspeicher oder über LAN bzw. WLAN bereitgestellt werden.

Dabei ist die Programmkomponente beispielsweise eine Datei, die Parametergrenzen und Plausibilitätsregeln definiert. Die Datei kann darüber hinaus auch Parameterquerabhängigkeiten definieren. Die Datei wird dann von der Computersoftware entsprechend ausgelesen und angewendet. Die Programmkomponente kann z.B. auch als eine funktionale Softwarekomponente, wie z.B. eine DLL-Datei, ausgebildet sein. Durch die von der Gerätesoftware gelieferte DLL-Datei wird die Computersoftware in die Lage versetzt, mit dem Beatmungsgerät 1 zu interagieren.

Durch die Programmkomponente wird somit eine Interaktion zwischen dem Computer 100 und dem Beatmungsgerät 1 trotz ungültiger Versionsstände ermöglicht. Dadurch kann die Computersoftware zuvor nicht mögliche oder zugängliche Funktionen bereitstellen und insbesondere das Beatmungsgerät 1 auslesen und ansteuern. Das ist besonders dann von Vorteil, wenn ein neuer Gerätetyp in den Markt gebracht wird, da die externen Computer 100 ohne die Bereitstellung der Programmkomponente durch die Gerätesoftware nicht in der Lage wären, das neue Gerät 1 zu unterstützen.

Ein zuverlässiger Betrieb des Beatmungssystems 100 wird auch dadurch erreicht, dass die Computersoftware im Entwicklungsprozess rückwärts kompatibel gehalten wird. Dadurch kann eine neue Computersoftware auch ältere Geräte 1 mit ungültiger Gerätesoftware unterstützen. Bei einem Aufbau einer Verbindung zwischen einem Computer 100 und einem Beatmungsgerät 1 werden z. B. Typ und Gerätesoftwareversion abgefragt und die Konfiguration entsprechend der Version durchgeführt. Sollte dabei erkannt werden, dass ein kritisches Update für die Gerätesoftware als zwingend notwendig definiert ist, wird dieses z.B. per Mitteilung an den Anwender angefordert.

Bei einem Auseinanderfallen der Versionsstände der Betriebsprogramme in einem Maß, welches den zuverlässigen Betrieb nicht mehr gewährleistet, kann eine Einforderung eines Updates vorgesehen sein. Dabei kann beispielsweise für die jeweils neuere Komponente, die Computersoftware und/oder die Gerätesoftware, definiert werden, dass diese das Zusammenspiel mit älteren Versionen der drei Betriebsprogramme sperrt und ein Update der jeweils älteren bzw. ungültigen Betriebsprogramme einfordert.

Für den Fall, dass eine Vielzahl von Geräten 1 gleich konfiguriert werden soll, ist ein Masterprogramm vorgesehen. Das Masterprogramm ist beispielsweise auf einer Speicherkarte hinterlegt. Damit kann eine Gerätekonfiguration oder z. B. eine Basiskonfiguration schnell und einfach auf einer Vielzahl von Geräten 1 hinterlegt werden. Da es in einem solchen Fall besonders darauf ankommt, dass keine Versionskonflikte auftreten, kann die Ausführung des Masterprogramms bei Vorliegen ungültiger Versionsstände blockiert werden.

Das Beatmungsgerät ist dabei z.B. ein BiLevel-Gerät, welches zunächst in einem S/T-Modus mit IPAP und EPAP betrieben wird und eine Vielzahl weiterer Parameter eingestellt hat. Bei einem Geräteupdate können EPAP und die weiteren Parameter unverändert übernommen werden. Hinzu kommt z.B. ein weiterer Gerätealarm. Für diesen kann die Werkseinstellung ("deaktiv") in die neue Gerätekonfiguration übernommen werden.

Weiterhin kommt z.B. eine Targetvolumenfunktion hinzu. D.h. zukünftig müssen im S/T-Modus IPAPmax und IPAPmin anstatt IPAP eingestellt werden, und zusätzlich das Targetvolumen VTarget. Für IPAPmax, IPAPmin und VTarget gibt es keine allgemeingültige Voreinstellung, diese müssen für den zu Beatmenden individuell ausgewählt werden. Diesen Konflikt erkennt die Gerätesoftware beim Update mit Hilfe der Anpassung der Gerätekonfiguration bzw. mit Hilfe der Programmkomponente. Sie fragt den Benutzer, z. B. den Arzt oder Techniker, nach den zu verwendenden Werten für IPAPmax, IPAPmin und VTarget für den aktuell zu Beatmenden. Über die Programmkomponente kennt sie den gültigen Wertebereich der neuen Parameter sowie die Querbeziehung IPAPmax >= IPAPmin. Dadurch wird die Auswahlmöglichkeit des Benutzers sinnvoll eingeschränkt. Erfolgt die Einstellung beim Update oder zu einem späteren Zeitpunkt über eine Computersoftware, so lernt diese über die Programmkomponente die Wertegrenzen und Querbeziehung der neuen Parameter. Obwohl die Targetvolumenfunktion zum Zeitpunkt der Erstellung der Computersoftware noch nicht vorhanden war, kann das Gerät dennoch korrekt über die Computersoftware eingestellt werden.

Die hier vorgestellten Verfahren und Vorrichtungen haben den Vorteil, dass möglichst viele Kombinationen verschiedener Versionsstände von Computersoftware und/oder Gerätesoftware und/oder Gerätekonfigurationen miteinander verwendbar sind. Dadurch können nach einem Update möglichst viele Benutzer- und Beatmungseinstellungen übernommen werden. Zudem ist dadurch gewährleistet, dass die resultierende Beatmungsfunktion sicher und definiert ist.

### Bezugszeichenliste:

- 1: Beatmungsgerät
- 2: Beatmungseinrichtung
- 3: Gebläseeinrichtung
- 4: Atemschnittstelle
- 5: Überwachungseinrichtung
- 6: Befeuchteeinrichtung
- 7: Bedieneinrichtung
- 8: Rechnereinrichtung
- 11: Heimbeatmungsgerät
- 10: Computer
- 14: Atemmaske
- 15: Speichereinrichtung
- 17: Anzeigeeinrichtung
- 24: Kopfhaube
- 25: Controller
- 27: Schnittstelle
- 34: Kupplungselement
- 35: Sensoreinrichtung
- 44: Ausatmungselement
- 54: Verbindungsschlauch
- 64: Koppeleinrichtung
- 100: Beatmungssystem
- 101: Gehäuse
- 350: Druckmessschlauch
- 351: Eingangsstutzen

## Patentansprüche

1. Verfahren zum Betreiben wenigstens eines Beatmungsgerätes (1) mit wenigstens einer Rechnereinrichtung (8) zur Steuerung einer Mehrzahl von Gerätefunktionen umfassend wenigstens eine Gerätesoftware und wenigstens eine Gerätekonfiguration mit Einstellparametern, wobei eine Gültigkeit der Gerätesoftware und eine Gültigkeit der Gerätekonfiguration jeweils durch wenigstens einen Versionsstand charakterisiert sind und wobei die Versionsstände zur Erkennung eines Versionskonfliktes zwischen der Gerätesoftware und der Gerätekonfiguration wenigstens teilweise miteinander verglichen werden, **dadurch gekennzeichnet, dass** bei einem ungültigen Versionsstand der Gerätekonfiguration und einem gültigen Versionsstand der Gerätesoftware die Einstellparameter der Gerätekonfiguration ausgelesen und in wenigstens eine erste Gruppe mit vom Versionskonflikt nicht betroffenen und wenigstens eine zweite Gruppe mit vom Versionskonflikt betroffenen Einstellparametern sortiert werden und dass die Einstellparameter der ersten Gruppe beibehalten werden, wobei die Einstellparameter Werte vorgeben, mit denen die für die Beatmung einzustellenden Beatmungsparameter auf bestimmte Werte und/oder Verläufe festgelegt werden, und wobei die Einstellparameter der ersten Gruppe von der Rechnereinrichtung (8) zur Steuerung angewendet werden, wobei die Einstellparameter der zweiten Gruppe in wenigstens eine erste Klasse mit für einen vorgesehenen Betrieb des Beatmungsgerätes (1) unkritischen und wenigstens eine zweite Klasse mit für einen vorgesehenen Betrieb des Beatmungsgerätes (1) kritischen Einstellparametern sortiert werden, wobei wenigstens ein Einstellparameter der ersten Klasse anhand wenigstens einer in der Gerätesoftware hinterlegten Vorschrift neu berechnet wird.

2. Verfahren nach dem vorhergehenden Anspruch, wobei die Vorschrift zur Berechnung eine Berücksichtigung wenigstens eines anderen Einstellparameters der ersten Gruppe und/oder der zweiten Gruppe vorsieht.

3. Verfahren nach einem der zwei vorhergehenden Ansprüche, wobei wenigstens ein Einstellparameter der ersten Klasse anhand wenigstens einer Benutzereingabe eingestellt wird.

4. Verfahren nach einem der drei vorhergehenden Ansprüche, wobei wenigstens ein Einstellparameter der ersten Klasse durch wenigstens eine in einer Speichereinrichtung (15) hinterlegte Werkseinstellung ersetzt wird.

5. Verfahren nach einem der vier vorhergehenden Ansprüche, wobei bei einem Vorliegen wenigstens eines Einstellparameters der zweiten Klasse wenigstens ein Hinweis über den Versionskonflikt an den Benutzer ausgegeben wird.

6. Verfahren nach einem der fünf vorhergehenden Ansprüche, wobei bei einem Vorliegen wenigstens eines Einstellparameters der zweiten Klasse nach Einforderung wenigstens einer Benutzereingabe ein vorheriger und gültiger Versionsstand der Gerätesoftware wiederhergestellt wird.

7. Verfahren nach einem der sieben vorhergehenden Ansprüche, wobei bei einem Vorliegen wenigstens eines Einstellparameters der zweiten Klasse wenigstens eine Benutzereingabe für einen Betrieb mit dem ungültigen Versionsstand aus Gerätesoftware und Gerätekonfiguration eingefordert wird und nach der Benutzereingabe der Betrieb mit dem ungültigen Versionsstand in einem wenigstens teilweise eingeschränkten Modus fortgesetzt wird.

8. Beatmungsgerät (1) mit wenigstens einer Rechnereinrichtung (8) zur Steuerung einer Mehrzahl von Gerätefunktionen umfassend wenigstens eine Gerätesoftware und wenigstens eine Gerätekonfiguration mit Einstellparametern, wobei eine Gültigkeit der Gerätesoftware und eine Gültigkeit der Gerätekonfiguration jeweils durch wenigstens einen Versionsstand charakterisiert sind und wobei die Rechnereinrichtung (8) dazu geeignet und ausgebildet ist, die Versionsstände zur Erkennung eines Versionskonfliktes zwischen der Gerätesoftware und der Gerätekonfiguration wenigstens teilweise miteinander zu vergleichen, **dadurch gekennzeichnet, dass** die Rechnereinrichtung (8) dazu geeignet und ausgebildet ist, wenigstens einen ungültigen Versionsstand der Gerätekonfiguration und wenigstens einen gültigen Versionsstand der Gerätesoftware zu registrieren und die Einstellparameter der Gerätekonfiguration auszulesen und in wenigstens eine erste Gruppe mit vom Versionskonflikt nicht betroffenen und wenigstens eine zweite Gruppe mit vom Versionskonflikt betroffenen Einstellparametern zu sortieren und die Einstellparameter der ersten Gruppe beizubehalten wobei die Einstellparameter Werte vorgeben, mit denen die für die Beatmung einzustellenden Beatmungsparameter auf bestimmte Werte und/oder Verläufe festgelegt werden, und wobei die Einstellparameter der ersten Gruppe von der Rechnereinrichtung (8) zur Steuerung angewendet werden, wobei die Einstellparameter der zweiten Gruppe in wenigstens eine erste Klasse, mit für einen vorgesehenen Betrieb des Beatmungsgerätes (1) unkritischen und wenigstens eine zweite Klasse, mit für einen vorgesehenen Betrieb des Beatmungsgerätes (1) kritischen Einstellparametern sortiert werden, wobei wenigstens ein Einstellparameter der ersten Klasse anhand wenigstens einer in der Gerätesoftware hinterlegten Vorschrift neu berechnet wird.

## Claims

1. A method for operating at least one artificial respiration device (1) with at least one computing apparatus (8) for controlling a multiplicity of device functions, comprising at least one piece of device software and at least one device configuration with adjustment parameters, wherein the validity of the device software and the validity of the device configuration are in each case **characterized by** at least one version and wherein the versions are at least partially compared with one another in order to recognize a version conflict between the device software and the device configuration, **characterized in that** in the event of an invalid version of the device configuration and a valid version of the device software, the adjustment parameters of the device configuration are read out and sorted into at least one first group having adjustment parameters which are not affected by the version conflict and at least one second group having adjustment parameters which are affected by the version conflict and the adjustment parameters of the first group are retained, wherein the adjustment parameters specify values, with which the artificial respiration parameters to be adjusted for the artificial respiration are fixed at specific values and/or courses, and wherein the adjustment parameters of the first group are applied by the computing apparatus (8) for control purposes, wherein the adjustment parameters of the second group are sorted into at least one first class with adjustment parameters which are uncritical for an envisaged operation of the artificial respiration device (1) and at least one second class with adjustment parameters which are critical for an envisaged operation of the artificial respiration device (1), wherein at least one adjustment parameter of the first class is recalculated on the basis of at least one instruction stored in the device software.

2. The method according to the preceding claim, wherein the calculation instruction provides for considering at least one other adjustment parameter of the first group and/or of the second group.

3. The method according to one of the two preceding claims, wherein at least one adjustment parameter of the first class is adjusted on the basis of at least one user input.

4. The method according to one of the three preceding claims, wherein at least one adjustment parameter of the first class is replaced by at least one factory adjustment stored in a storage apparatus. (15).

5. The method according to one of the four preceding claims, wherein, in the event of at least one adjustment parameter of the second class existing, at least one indication regarding the version conflict is output to the user.

6. The method according to one of the five preceding claims, wherein, in the event of at least one adjustment parameter of the second class existing, in accordance with the requirement of at least one user input, a previous and valid version of the device software is restored.

7. The method according to one of the seven preceding claims, wherein, in the event of at least one adjustment parameter of the second class existing, at least one user input is required for operation with the invalid version of the device software and device configuration and, following the user input, the operation is continued with the invalid version in an at least partially restricted mode.

8. An artificial respiration device (1) having at least one computing apparatus (8) for controlling a multiplicity of device functions, comprising at least one piece of device software and at least one device configuration with adjustment parameters, wherein the validity of the device software and the validity of the device configuration are in each case **characterized by** at least one version and wherein the computing apparatus (8) is suitable and designed for at least partially comparing the versions with one another in order to recognize a version conflict between the device software and the device configuration, **characterized in that** the computing apparatus (8) is suitable and designed for registering at least one invalid version of the device configuration and at least one valid version of the device software and for reading out the adjustment parameters of the device configuration and sorting these into at least one first group having adjustment parameters which are not affected by the version conflict and at least one second group having adjustment parameters which are affected by the version conflict and for retaining the adjustment parameters of the first group, wherein the adjustment parameters specify values, with which the artificial respiration parameters to be adjusted for the artificial respiration are fixed at specific values and/or courses, and wherein the adjustment parameters of the first group are applied by the computing apparatus (8) for control purposes, wherein the adjustment parameters of the second group are sorted into at least one first class with adjustment parameters which are uncritical for an envisaged operation of the artificial respiration device (1) and at least one second class with adjustment parameters which are critical for an envisaged operation of the artificial respiration device (1), wherein at least one adjustment parameter of the first class is recalculated on the basis of at least one instruction stored in the software.

## Revendications

1. Procédé de fonctionnement d'au moins un appareil respiratoire (1) doté d'au moins un dispositif informatique (8) destiné à commander une pluralité de fonctions d'appareil comprenant au moins un logiciel d'appareil et au moins une configuration d'appareil avec des paramètres de réglage, dans lequel une validité du logiciel d'appareil et une validité de la configuration d'appareil se caractérisent respectivement par au moins un statut de version et dans lequel les statuts de version sont au moins partiellement comparés entre eux afin de détecter un conflit de versions entre le logiciel d'appareil et la configuration d'appareil, **caractérisé en ce qu'en** cas de statut de version invalide de la configuration d'appareil et de statut de version valide du logiciel d'appareil, les paramètres de réglage de la configuration d'appareil sont lus et classés en au moins un premier groupe comprenant des paramètres de réglage non concernés par le conflit de versions et au moins un deuxième groupe comprenant des paramètres de réglage concernés par le conflit de versions et **en ce que** les paramètres de réglage du premier groupe sont conservés, dans lequel les paramètres de réglage indiquent des valeurs avec lesquelles les paramètres respiratoires à régler pour la respiration sont fixés à des valeurs et/ou des évolutions déterminés, et dans lequel les paramètres de réglage du premier groupe sont utilisés par le dispositif informatique (8) pour la commande, dans lequel les paramètres de réglage du deuxième groupe sont classés en au moins une première catégorie comprenant des paramètres de réglage non critiques pour un fonctionnement prévu de l'appareil respiratoire (1) et au moins une deuxième catégorie comprenant des paramètres de réglage critiques pour un fonctionnement prévu de l'appareil respiratoire (1), dans lequel au moins un paramètre de réglage de la première catégorie est calculé de nouveau à l'aide d'au moins une consigne enregistrée dans le logiciel d'appareil.

2. Procédé selon la revendication précédente, dans lequel la consigne de calcul prévoit une prise en compte d'au moins un autre paramètre de réglage du premier groupe et/ou du deuxième groupe.

3. Procédé selon l'une des deux revendications précédentes, dans lequel au moins un paramètre de réglage de la première catégorie est réglé à l'aide d'au moins une entrée d'utilisateur.

4. Procédé selon l'une des trois revendications précédentes, dans lequel au moins un paramètre dé réglage de la première catégorie est remplacé par au moins un réglage d'usine enregistré dans un dispositif de mémoire (15).

5. Procédé selon l'une des quatre revendications précédentes, dans lequel, en présence d'au moins un paramètre de réglage de la deuxième catégorie, au moins un avertissement concernant le conflit . de versions est délivré à l'utilisateur.

6. Procédé selon l'une des cinq revendications précédentes, dans lequel, en présence d'au moins un paramètre de réglage de la deuxième catégorie, après la réclamation d'au moins une entrée d'utilisateur, un statut de version précédent et valide du logiciel d'appareil est rétabli.

7. Procédé selon l'une des sept revendications précédentes, dans lequel, en présence d'au moins un paramètre de réglage de la deuxième catégorie, au moins une entrée d'utilisateur pour un fonctionnement avec le statut de version invalide du logiciel d'appareil et de la configuration d'appareil est demandée et le fonctionnement avec le statut de version invalide est poursuivi dans un mode au moins partiellement restreint après l'entrée d'utilisateur.

8. Appareil respiratoire (1) doté d'au moins un dispositif informatique (8) destiné à commander une pluralité de fonctions d'appareil comportant au moins un logiciel d'appareil et au moins une configuration d'appareil avec des paramètres de réglage, dans lequel une validité du logiciel d'appareil et une validité de la configuration d'appareil se caractérisent respectivement par au moins un statut de version et dans lequel le dispositif informatique (8) est adapté et conçu pour comparer au moins partiellement les statuts de version entre eux afin de détecter un conflit de versions entre le logiciel d'appareil et la configuration d'appareil, **caractérisé en ce que** le dispositif informatique (8) est adapté et conçu pour enregistrer au moins un statut de version invalide de la configuration d'appareil et au moins un statut de version valide du logiciel d'appareil et pour lire les paramètres de réglage de la configuration d'appareil et classés ceux-ci en au moins un premier groupe comprenant des paramètres de réglage non concernés par le conflit de versions et au moins un deuxième groupe comprenant des paramètres de réglage concernés par le conflit de versions et pour conserver les paramètres de réglage du premier groupe, dans lequel les paramètres de réglage indiquent des valeurs avec lesquelles les paramètres respiratoires à régler pour la respiration sont fixés à des valeurs et/ou des évolutions déterminés, et dans lequel les paramètres de réglage du premier groupe sont utilisés par le dispositif informatique (8) pour la commande, dans lequel les paramètres de réglage du deuxième groupe sont classés en au moins une première catégorie comprenant des paramètres de réglage non critiques pour un fonctionnement prévu de l'appareil respiratoire (1) et au moins une deuxième catégorie comprenant des paramètres de réglage critiques pour un fonctionnement prévu de l'appareil respiratoire (1), dans lequel au moins un paramètre de réglage de la première catégorie est calculé de nouveau à l'aide d'au moins une consigne enregistrée dans le logiciel d'appareil.
